# EUROPEAN PATENT APPLICATION

(11) **EP 3 922 726 A1**
(43) Date of publication of application: **15.12.2021**
(21) Application number: 20179361.9
(22) Date of filing: 10.06.2020
(51) Int. Cl.: C12N 15/81, C12P 1/02, C12R 1/73, C12R 1/78, C12R 1/84

(54) **TRUNCATED PROMOTER FOR RECOMBINANT GENE EXPRESSION**

(71) Applicant: Erber Aktiengesellschaft, 3131 Getzersdorf bei Traismauer (AT)
(72) Inventor: KRAINER, Florian, 3430 Tulln (AT); STURMBERGER, Lukas, 1020 Wien (AT); MENCZIK, Patricia, 1210 Wien (AT)
(74) Representative: Cunow, Gerda

(57) **Abstract**

The present invention relates to promoters with increased relative expression efficiency, in particular to promoter variants of a formate dehydrogenase promoter having increased relative expression efficiency, a method for recombinant protein production and for increasing the relative expression efficiency of a nucleotide sequence having promoter activity.

SEQUENCE LISTING

<110> Erber Aktiengesellschaft

<120> TRUNCATED PROMOTER FOR RECOMBINANT GENE EXPRESSION

<130> P06005

<160> 24

<170> BiSSAP 1.3.6

<210> 1
<211> 623
<212> DNA
<213> Ogataea polymorpha

<400> 1
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aatggcacta taaataccgc ctccttgcgc 600

tctctgcctt catcaatcaa atc 623

<210> 2
<211> 1000
<212> DNA
<213> Komagataella pastoris

<400> 2
   cgattgcccc tctacaggca taagggtgtg actttgtggg cttgaatttt acaccccctc 60
   caacttttct cgcatcaatt gatcctgtta ccaatattgc atgcccggag gagacttgcc 120
   ccctaatttc gcggcgtcgt cccggatcgc agggtgagac tgtagagacc ccacatagtg 180
   acaatgatta tgtaagaaga ggggggtgat tcggccggct atcgaactct aacaactagg 240
   ggggtgaaca atgcccagca gtcctcccca ctctttgaca aatcagtatc accgattaac 300
   accccaaatc ttattctcaa cggtccctca tccttgcacc cctctttgga caaatggcag 360
   ttagcattgg tgcactgact gactgcccaa ccttaaaccc aaatttctta gaaggggccc 420
   atctagttag cgaggggtga aaaattcctc catcggagat gtattgaccg taagttgctg 480
   cttaaaaaaa atcagttcag atagcgagac ttttttgatt tcgcaacggg agtgcctgtt 540
   ccattcgatt gcaattctca ccccttctgc ccagtcctgc caattgccca tgaatctgct 600
   aatttcgttg attcccaccc ccctttccaa ctccacaaat tgtccaatct cgttttccat 660
   ttgggagaat ctgcatgtcg actacataaa gcgaccggtg tccgaaaaga tctgtgtagt 720
   tttcaacatt ttgtgctccc cccgctgttt gaaaacgggg gtgagcgctc tccggggtgc 780
   gaattcgtgc ccaattcctt tcaccctgcc tattgtagac gtcaacccgc atctggtgcg 840
   aatatagcgc acccccaatg atcacaccaa caattggtcc acccctcccc aatctctaat 900
   attcacaatt cacctcacta taaatacccc tgtcctgctc ccaaattctt ttttccttct 960
   tccatcagct actagctttt atcttattta ctttacgaaa 1000

<210> 3
<211> 1000
<212> DNA
<213> Komagataella pastoris

<400> 3
atgttggtat tgtgaaatag acgcagatcg ggaacactga aaaataacag ttattattcg 60

agatctaaca tccaaagacg aaaggttgaa tgaaaccttt ttgccatccg acatccacag 120

gtccattctc acacataagt gccaaacgca acaggagggg atacactagc agcagaccgt 180

tgcaaacgca ggacctccac tcctcttctc ctcaacaccc acttttgcca tcgaaaaacc 240

agcccagtta ttgggcttga ttggagctcg ctcattccaa ttccttctat taggctacta 300

acaccatgac tttattagcc tgtctatcct ggcccccctg gcgaggttca tgtttgttta 360

tttccgaatg caacaagctc cgcattacac ccgaacatca ctccagatga gggctttctg 420

agtgtggggt caaatagttt catgttcccc aaatggccca aaactgacag tttaaacgct 480

gtcttggaac ctaatatgac aaaagcgtga tctcatccaa gatgaactaa gtttggttcg 540

ttgaaatgct aacggccagt tggtcaaaaa gaaacttcca aaagtcggca taccgtttgt 600

cttgtttggt attgattgac gaatgctcaa aaataatctc attaatgctt agcgcagtct 660

ctctatcgct tctgaacccc ggtgcacctg tgccgaaacg caaatgggga aacacccgct 720

ttttggatga ttatgcattg tctccacatt gtatgcttcc aagattctgg tgggaatact 780

gctgatagcc taacgttcat gatcaaaatt taactgttct aacccctact tgacagcaat 840

atataaacag aaggaagctg ccctgtctta aacctttttt tttatcatca ttattagctt 900

actttcataa ttgcgactgg ttccaattga caagcttttg attttaacga cttttaacga 960

caacttgaga agatcaaaaa acaactaatt attcgaaacg 1000

<210> 4
<211> 1000
<212> DNA
<213> Komagataella pastoris

<400> 4
aaatggcaga aggatcagcc tggacgaagc aaccagttcc aactgctaag taaagaagat 60

gctagacgaa ggagacttca gaggtgaaaa gtttgcaaga agagagctgc gggaaataaa 120

ttttcaattt aaggacttga gtgcgtccat attcgtgtac gtgtccaact gttttccatt 180

acctaagaaa aacataaaga ttaaaaagat aaacccaatc gggaaacttt agcgtgccgt 240

ttcggattcc gaaaaacttt tggagcgcca gatgactatg gaaagaggag tgtaccaaaa 300

tggcaagtcg ggggctactc accggatagc caatacattc tctaggaacc agggatgaat 360

ccaggttttt gttgtcacgg taggtcaagc attcacttct taggaatatc tcgttgaaag 420

ctacttgaaa tcccattggg tgcggaacca gcttctaatt aaatagttcg atgatgttct 480

ctaagtggga ctctacggct caaacttcta cacagcatca tcttagtagt cccttcccaa 540

aacaccattc taggtttcgg aacgtaacga aacaatgttc ctctcttcac attgggccgt 600

tactctagcc ttccgaagaa ccaataaaag ggaccggctg aaacgggtgt ggaaactcct 660

gtccagttta tggcaaaggc tacagaaatc ccaatcttgt cgggatgttg ctcctcccaa 720

acgccatatt gtactgcagt tggtgcgcat tttagggaaa atttacccca gatgtcctga 780

ttttcgaggg ctacccccaa ctccctgtgc ttatacttag tctaattcta ttcagtgtgc 840

tgacctacac gtaatgatgt cgtaacccag ttaaatggcc gaaaaactat ttaagtaagt 900

ttatttctcc tccagatgag actctccttc ttttctccgc tagttatcaa actataaacc 960

tattttacct caaatacctc caacatcacc cacttaaaca 1000

<210> 5
<211> 160
<212> DNA
<213> Artificial Sequence

<220>
<223> Synthetic promoter

<400> 5
cggggtgtct cactggtgtc cgccaataag aggagccggc aggcacggag tctacatcaa 60

gctgtctccg atacactcga ctaccatccg ggtctctcag agaggggaat ggcactataa 120

ataccgcctc cttgcgctct ctgccttcat caatcaaatc 160

<210> 6
<211> 623
<212> DNA
<213> Artificial Sequence

<220>
<223> Synthetic promoter

<400> 6
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagggggg aatggcacta taaataccgc ctccttgcgc 600

tctctgcctt catcaatcaa atc 623


<210> 7
<211> 623
<212> DNA
<213> Artificial Sequence


<220>
<223> Synthetic promoter

<400> 7
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccag ccgggtctct cagagagggg aatggcacta taaataccgc ctccttgcgc 600

tctctgcctt catcaatcaa atc 623


<210> 8

<211> 623
<212> DNA
<213> Artificial Sequence

<220>
<223> Synthetic promoter

<400> 8
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct ccgagagggg aatggcacta taaataccgc ctccttgcgc 600

tctctgcctt catcaatcaa atc 623

<210> 9
<211> 623
<212> DNA
<213> Artificial Sequence

<220>
<223> Synthetic promoter

<400> 9
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cacagagggg aatggcacta taaataccgc ctccttgcgc 600

tctctgcctt catcaatcaa atc 623

<210> 10
<211> 623
<212> DNA
<213> Artificial Sequence

<220>
<223> Synthetic promoter

<400> 10
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagcgagggg aatggcacta taaataccgc ctccttgcgc 600

tctctgcctt catcaatcaa atc 623

<210> 11
<211> 623
<212> DNA
<213> Artificial Sequence

<220>
<223> Synthetic promoter

<400> 11
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagacagggg aatggcacta taaataccgc ctccttgcgc 600

tctctgcctt catcaatcaa atc 623

<210> 12
<211> 623
<212> DNA
<213> Artificial Sequence

<220>
<223> Synthetic promoter

<400> 12
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagcgggg aatggcacta taaataccgc ctccttgcgc 600

tctctgcctt catcaatcaa atc 623

<210> 13
<211> 623
<212> DNA
<213> Artificial Sequence

<220>
<223> Synthetic promoter

<400> 13
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagacggg aatggcacta taaataccgc ctccttgcgc 600

tctctgcctt catcaatcaa atc 623

<210> 14
<211> 623
<212> DNA
<213> Artificial Sequence

<220>
<223> Synthetic promoter

<400> 14
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagcgg aatggcacta taaataccgc ctccttgcgc 600

tctctgcctt catcaatcaa atc 623

<210> 15
<211> 623
<212> DNA
<213> Artificial Sequence

<220>
<223> Synthetic promoter

<400> 15
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagaggcg aatggcacta taaataccgc ctccttgcgc 600

tctctgcctt catcaatcaa atc 623

<210> 16
<211> 623
<212> DNA
<213> Artificial Sequence

<220>
<223> Synthetic promoter

<400> 16
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggc aatggcacta taaataccgc ctccttgcgc 600

tctctgcctt catcaatcaa atc 623

<210> 17
<211> 623
<212> DNA
<213> Artificial Sequence

<220>
<223> Synthetic promoter

<400> 17
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg catggcacta taaataccgc ctccttgcgc 600

tctctgcctt catcaatcaa atc 623

<210> 18
<211> 623
<212> DNA
<213> Artificial Sequence

<220>
<223> Synthetic promoter

<400> 18
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg actggcacta taaataccgc ctccttgcgc 600

tctctgcctt catcaatcaa atc 623

<210> 19
<211> 623
<212> DNA
<213> Artificial Sequence

<220>
<223> Synthetic promoter

<400> 19
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aacggcacta taaataccgc ctccttgcgc 600

tctctgcctt catcaatcaa atc 623

<210> 20
<211> 623
<212> DNA
<213> Artificial Sequence

<220>
<223> Synthetic promoter

<400> 20
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aatcgcacta taaataccgc ctccttgcgc 600

tctctgcctt catcaatcaa atc 623

<210> 21
<211> 623
<212> DNA
<213> Artificial Sequence

<220>
<223> Synthetic promoter

<400> 21
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aatgccacta taaataccgc ctccttgcgc 600

tctctgcctt catcaatcaa atc 623

<210> 22
<211> 648
<212> DNA
<213> Artificial Sequence

<220>
<223> Synthetic promoter

<400> 22
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aatggcacta tataaactgg tgataattcc 600

ttcgttctga gttccatctc atactcaaac tatattaaaa ctacaaca 648

<210> 23
<211> 639
<212> DNA
<213> Artificial Sequence

<220>
<223> Synthetic promoter

<400> 23

aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aatggcacta taaatacaag acgagtgcgt 600

ccttttctag actcacccat aaacaaataa tcaataaat 639

<210> 24
<211> 623
<212> DNA
<213> Artificial Sequence

<220>
<223> Synthetic promoter

<400> 24
aatgtatcta aacgcaaact ccgagctgga aaaatgttac cggcgatgcg cggacaattt 60

agaggcggcg atcaagaaac acctgctggg cgagcagtct ggagcacagt cttcgatggg 120

cccgagatcc caccgcgttc ctgggtaccg ggacgtgagg cagcgcgaca tccatcaaat 180

ataccaggcg ccaaccgagt ctctcggaaa acagcttctg gatatcttcc gctggcggcg 240

caacgacgaa taatagtccc tggaggtgac ggaatatata tgtgtggagg gtaaatctga 300

cagggtgtag caaaggtaat attttcctaa aacatgcaat cggctgcccc gcaacgggaa 360

aaagaatgac tttggcactc ttcaccagag tggggtgtcc cgctcgtgtg tgcaaatagg 420

ctcccactgg tcaccccgga ttttgcagaa aaacagcaag ttccggggtg tctcactggt 480

gtccgccaat aagaggagcc ggcaggcacg gagtctacat caagctgtct ccgatacact 540

cgactaccat ccgggtctct cagagagggg aatggcacta taaatactgc ctacttgtcc 600

tctattcctt catcaatcac atc 623

## Description

The present invention relates to a truncated promoter or variant thereof, a method for recombinant production of a target polypeptide and to a method for increasing the relative expression efficiency of a nucleotide sequence having promoter activity.

The use of specific nucleotide sequences as promoters to facilitate recombinant gene expression and to achieve target protein production has been studied in the field of biotechnology for decades. Promoters for gene expression in prokaryotic hosts are typically relatively short in length, often consisting of less than 100 base pairs (bp), such as the T7 RNA polymerase promoter or the promoter of the *E. coli lac* operon. On the other hand, promoters for gene expression in eukaryotic hosts are typically between 500 and 1000 bp long, such as the *FLD1* promoter or the *AOX1* promoter of *Pichia pastoris,* and even promoters of more than 1 kb (kilo base pairs) are not uncommon.

Typical prokaryotic host organisms for recombinant gene expression are *Escherichia coli* or *Bacillus subtilis.* Examples for eukaryotic host organisms are *Saccharomyces cerevisiae*, *Trichoderma reesei* or *Pichia pastoris.* In the last years, strains of the yeast *P*. *pastoris* have been established as recombinant production platforms for industrially important proteins.

Well-known promoters to regulate recombinant gene expression in *P*. *pastoris* are the methanol-inducible promoter of the alcohol oxidase 1 gene (*AOX1*) and the constitutive promoter of the glyceraldehyde-3-phosphate dehydrogenase gene (*GAP*)*.* Hartner et al. performed promoter engineering studies on the *P*. *pastoris* alcohol oxidase 1 promoter to identify transcription factor binding sites or to verify putative transcription factor binding sites by targeted short internal deletions within the *P*. *pastoris* alcohol oxidase 1 promoter nucleotide sequence (Hartner et al. Nucleid Acids Res. 2008. 36(12:e76)). In addition, in recent years, several new promoter sequences with different kinds of regulation and expression strengths were described (e.g. Vogl et al. ACS Synth Biol. 2016. 5(2): 172-186). Despite these scientific advancements, rational prediction of essential elements in a eukaryotic promoter is not yet reliable. Essential regulatory elements are often separated from one another by hundreds or even thousands of base pairs on the primary nucleotide sequence and may only interact due to secondary or tertiary nucleotide structures. As a consequence, the effects of insertions or deletions of nucleotide sequence stretches cannot be predicted. Therefore, eukaryotic promoter engineering endeavours still rely largely on intensive experimentation with so far unpredictable outcome.

In EP 0 299 108 A1, a nucleotide sequence is disclosed comprising control regions and a structural gene encoding a formate dehydrogenase enzyme.

In WO 03/095653 A1, nucleotide sequences are disclosed of engineered variants of the promoter of the formate dehydrogenase gene (*FMD*) from *Ogataea angusta.* The disclosed invention relates to promoter variants comprising palindromized nucleotide sequences to achieve higher transcription rates.

In WO 2017/109082 A1, variants of a formate dehydrogenase promoter for recombinant gene expression in a *Komagataella sp.* yeast cell are disclosed, which variants have a length of approximately 620 bp and are at least 90% identical to a non-mutated, wildtype *FMD* promoter of *Ogataea angusta*, also known as *Ogataea polymorpha* or *Hansenula polymorpha.* These variants illustrate the extent of single point mutations or deletions or insertions that can be introduced into this nucleotide sequence without negatively affecting its promoter function. It is emphasized that the shown promoters are not only inducible by methanol, but can be beneficially induced by so-called derepression. Induction by derepression is described to be achieved by depletion of a repressing carbon source such as glucose or glycerol or by feeding a non-repressing carbon source such as sorbitol instead. Remarkably, particularly high induction of the wildtype *FMD* promoter is shown when the glucose feed was ceased entirely.

In Song et al. (Bioechnol Lett. 2003. 25(23): 1999-2006), expression vectors comprising either a 645 bp variant of the *FMD* promoter or a methanol oxidase promoter of *Hansenula polymorpha* are described for the production of green fluorescent protein as reporter protein in *H*. *polymorpha.*

In Vogl et al. (AMB Express. 2020. 10(1): 38), a 623 bp wildtype *FMD* promoter of *H*. *polymorpha* is described to enable strong derepressed expression in *P*. *pastoris.* In particular, it is emphasized that derepression leads to strong activation of the *HpFMD* promoter in *P*. *pastoris* when the repressing carbon source, such as glucose or glycerol, is depleted.

In the workflow required to achieve recombinant gene expression, one essential step is the amplification of the expression plasmid and in particular the expression cassette. Plasmid amplification is typically performed in bacterial cells, such as *E. coli.* After isolation of the amplified plasmid from the cells, an expression cassette may be excised from the circular plasmid via restriction enzyme digest. Alternatively, expression cassettes may be obtained by DNA amplification via polymerase chain reaction (PCR). Notably, both strategies rely on DNA replication by DNA polymerases, which are prone to introducing undesired mutations into the expression cassette. Despite improvements made on *E. coli* strains for plasmid amplification as well as on polymerase enzymes for PCR, a certain number of mutated expression cassette molecules has to be accounted for in any amplicifation step and thus cannot be excluded. Statistically, DNA polymerases produce an erroneous nucleotide sequence after a certain number of nucleotides processed. As a consequence, the longer an expression cassette, the higher will be the risk of an introduction of unwanted mutations during DNA replication by polymerases. Also, the yield of host cells successfully transformed with an expression cassette benefits from higher numbers of molecules. However, PCRs are known to yield less product, i.e. fewer molecules, when the nucleotide sequence to be amplified is long. Therefore, increased PCR product yields may be achieved by reducing the length of the nucleotide sequence to be amplified. It is thus desirable to use an expression cassette that is as short as possible while still fulfilling its purpose. In general, the molecular assembly of a gene expression cassette or a plasmid is known to be achieved with higher success rates when the individual polynucleotide fragments to be assembled are shorter in length. Also in this regard, it is thus desirable to work with shorter fragments rather than with longer fragments. However, fragment shortening by truncation of functional polynucleotide sequences such as promoters cannot be expected not to negatively affect the activity of such a functional sequence. In particular, the effect on the functionality of a eukaryotic promoter upon omission of stretches of nucleotides of the promoter sequence is unpredictable to date (Hartner et al. Nucleid Acids Res. 2008. 36(12:e76)).

It is therefore an objective of the present invention to provide more efficient eukaryotic promoters that possess high expression strength at short length, i.e. eukaryotic promoters with increased relative expression efficiency.

This objective is achieved by providing a promoter, wherein the promoter is truncated from the 5'-end of the nucleotide sequence of SEQ ID NO: 1 to a length of 140-610 base pairs, preferably to a length of 140-560 base pairs, more preferably to a length of 160-560 base pairs, most preferably to a length of 160-500 base pairs; or by providing a variant thereof, i.e. a promoter variant, wherein the promoter variant has at least 80% identity, preferably at least 90% identity, preferably at least 95% identity, more preferably at least 97% identity, most preferably at least 99% identity to the promoter. A skilled person will appreciate the surprising effect that despite shortening of the overall length of the naturally occurring promoter having the nucleotide sequence of SEQ ID NO: 1 or variants thereof, the activity of a truncated promoter or promoter variant as described herein remains non-eliminated. By providing such a truncated or shortened promoter or promoter variant, it becomes possible to even use short synthetic oligonucleotides, such as primer oligonucleotides, that can be added to other recombinant nucleotide fragments in a simple PCR reaction, thus substantially accelerating the steps required for the assembly of recombinant gene expression cassettes, while reducing the risk for introducing point mutations - as is the case when having to rely on the assembly of larger polynucleotide fragments.

It is common practice in the art to write deoxyribonucleic acid (DNA) sequences, in particular promoter sequences in 5'-3' orientation. The 5'-end refers to the 5'-carbon atom of the ribose part of a nucleotide, whereas the 3'-end refers to the 3'-carbon atom. For the avoidance of any doubt, the 5'-end of a nucleotide sequence of a promoter is the distal end of a nucleotide sequence of a promoter. In other words, the 5'-end is determined by the nucleotide farthest away from the start codon of an open reading frame whose transcription is regulated by the promoter. The 5'-end of a nucleotide sequence of a promoter is the most upstream nucleotide from the start codon of an open reading frame whose transcription is regulated by the promoter. In contrast, the 3'-end of a nucleotide sequence of a promoter is the closest nucleotide upstream of the start codon of an open reading frame whose transcription is regulated by the promoter. In other words, the 3'-end of a nucleotide sequence of a promoter is the last nucleotide of a promoter upstream of the start codon of an open reading frame whose transcription is regulated by the promoter.

A person having skill in the art is aware of the fact, that small numbers of single point mutations, single nucleotide insertions or single nucleotide deletions can be introduced into a promoter nucleotide sequence without substantially lowering the activity or function of the promoter nucleotide sequence. The activity or function of a promoter would be considered to be substantially lowered when one or more single point mutation(s), insertion(s) or deletion(s) would cause a change in function by at least 60% compared to the function of the unaltered promoter. The present invention therefore also encompasses mutant promoter variants having at least 80% identity, preferably at least 90% identity, more preferably at least 95% identity, more preferably at least 97% identity, most preferably at least 99% identity to a truncated promoter as described herein. Merely for the sake of clarity it is noted, that promoters or promoter variants, which promoters or promoter variants are truncated from the 5'-end of the nucleotide sequence of SEQ ID NO: 1 to a length of more than 610 base pairs (e.g. promoters or promoter variants having 611 bp, 612 bp, 620 bp or even longer) are not encompassed by the present invention. Even though such longer promoters or promoter variants comprise promoter sequences of less than 610 bp, these promoters or promoter variants having a length of more than 610 base pairs are not encompassed by the present invention since their relative expression efficiency is not considered increased sufficiently high. In line with this, also promoter variants having at least 80% identity, preferably at least 90% identity, more preferably at least 95% identity, more preferably at least 97% identity, most preferably at least 99% identity to a truncated promoter as described herein, and having a length of more than 610 base pairs, are not encompassed by the present invention. Also accordingly, the non-truncated promoter having the nucleotide sequence of SEQ ID NO: 1 is not encompassed by the present invention, even though it comprises promoters having less than 610 bp. In other words, a promoter or a promoter variant according to the present invention does not occur in nature, but requires at least a step of truncation by an experimenter. Thus, a promoter or promoter variant according to the present invention relates exclusively to non-natural or recombinant, man-made molecules.

A person having skill in the art knows methods to determine the degree of sequence identity in percent (%) between two or more nucleotide sequences. As an example, the Needleman-Wunsch algorithm may be used (Needleman, Wunsch. J Mol Biol. 1970. 48(3):443-453). Appropriate tools are available online, e.g. from NCBI (National Center for Biotechnology Information; https:/Iblast.ncbi.nlm.nih.gov/Blast.cgi?PAGE_TYPE=BlastSearch&PROG_DEF=blastn&BLAST _PROG_DEF=blastn&BLAST_SPEC=GlobalAIn&LINK_LOC=BlastHomeLink) using default Algorithm parameters (Match/Mismatch Scores: 2,-3. Gap Costs: Existence: 5 Extension: 2). It is considered that a global alignment algorithm such as the Clustal Omega tool from the European Bioinformatics Institute shall be used (https://www.ebi.ac.uk/Tools/msa/clustalo/) using the default settings for DNA input sequences. When determining the degree of sequence identity between a promoter and a promoter variant comprising point mutations and/or point insertions and/or point deletions, using the Needleman-Wunsch algorithm or a comparable global alignment algorithm, only corresponding nucleotide sequences, i.e. nucleotide sequences having the same length shall be compared. For instance, in case a promoter variant is truncated from the 5'-end of the nucleotide sequence of SEQ ID NO: 1 to a length of 160 bp and this promoter variant further comprises three point mutations, the corresponding promoter sequence to which the promoter variant shall be aligned, shall also have a length of 160 bp. In other words, in order to allow correct determination of the degree of sequence identity in percent between a sequence segment comprised in SEQ ID NO: 1 and one or more promoter variants, the length of the sequence segment of SEQ ID NO: 1 shall be identical to the length of the promoter variant. Only then will the nucleotide sequence segment of SEQ ID NO: 1 be the correct corresponding nucleotide sequence segment of SEQ ID NO: 1. Thus, only in case the promoter variant does not comprise any point mutation(s) or insertion(s) or deletion(s), it will be 100% identical to a corresponding nucleotide sequence segment of SEQ ID NO: 1 in a global alignment.

In a preferred embodiment of the present invention, the promoter or promoter variant as described herein has a relative expression efficiency which is at least 5%, preferably at least 10% higher than the relative expression efficiency of the promoter having the nucleotide sequence of SEQ ID NO: 1. Hereby, it can be achieved that the expression strength of a truncated promoter or promoter variant is at least as high as the expression strength of a non-truncated promoter in relation to its length.

The term "relative expression efficiency" as used herein refers to the amount of target protein produced per base pair of promoter nucleotide sequence. In case a target protein is an enzyme, "relative expression efficiency" (REE) may refer to the amount of enzyme activity per base pair of promoter nucleotide sequence. For instance, if expression by a non-truncated promoter having 623 bp yields 80 units of enzyme activity per liter (U/L), its relative expression efficiency would be 80 / 623 = 0.128 U/L/bp. If expression by a truncated promoter having 500 bp yields 83 U/L, its relative expression efficiency would be 83 / 500 = 0.166 U/L/bp. The increase of the "relative expression efficiency" of the truncated promoter over the non-truncated promoter in percent can be calculated by multiplying the relative expression efficiency of the truncated promoter variant with 100 and dividing by the relative expression efficiency of the non-truncated promoter: 0.166 * 100 / 0.128 = 129.3%. Thus, the truncated promoter having 500 bp would have a relative expression efficiency that is 29.3% higher than the relative expression efficiency of the promoter having 623 bp. In other words, the truncated promoter having 500 bp would have an 1.293-fold higher relative expression efficiency than the promoter having 623 bp.

For the avoidance of doubt, a truncated promoter is considered to have a higher REE than a non-truncated promoter when a higher REE is determined from either a small scale cultivation such as cultivation in a deep well plate, or a medium scale cultivation such as cultivation in a shake flask, or a large scale cultivation such as cultivation in a bioreactor. It is well known in the art that transformation of yeasts such as *Pichia pastoris* typically results in a large number of transformant strains, which transformant strains do not perform identically, e.g. Vogl et al. (Appl Environ Microbiol. 2018. 84(6): e02712-17). When comparing different expression strategies, such as the comparison of different promoter variants, it is thus necessary to perform an initial screening of a plurality of transformants to exclude obvious non-performing strains or obvious poorly performing strains. Without wishing to be bound by theory, it is believed that such non- or poorly performing strains are the result of destructive genomic integration, where an expression cassette is integrated into a genomic locus that destroys or at least reduces the expression capability of the expression cassette. It is thus necessary to select only strains for comparison to one another, which strains are representative of the respective expression strategy. In other words, care needs to be taken to only compare strains harboring the same amount of integrated expression cassettes, as well as to compare at least 5, preferably at least 6, more preferably at least 7, even more preferably at least 8, most preferably at least 9 transformant strains out of 90 transformants from each expression strategy.

The present invention further relates to a promoter or promoter variant as described herein, wherein the promoter or promoter variant is comprised in an expression cassette, wherein the expression cassette further comprises a nucleotide sequence coding for a polypeptide and optionally a transcription terminator sequence, and wherein the promoter or promoter variant is operably linked to the nucleotide sequence coding for a polypeptide. Just for the sake of clarity, also the optional transcription terminator sequence is operably linked to the nucleotide sequence coding for a polypeptide. Surprisingly, by providing such an expression cassette it becomes possible to achieve recombinant gene expression at the same level as a non-truncated promoter while having a smaller molecule size due to the reduced promoter length. Consequently, when transforming cells of a production host organism with a certain amount of DNA of such an expression cassette, more expression cassette molecules can be introduced into the cells than when transforming a production host organism with the same amount of DNA of an expression cassette comprising a non-truncated, thus longer promoter. In a preferred embodiment of the invention, the promoter or promoter variant as described herein is operably linked to a recombinant nucleotide sequence coding for a heterologous polypeptide. Hereby, it becomes possible to produce proteins that are normally not produced by a host cell in nature. The present invention further relates to a promoter or promoter variant as described herein, wherein the promoter or promoter variant is comprised in a plasmid, and wherein the promoter or promoter variant is operably linked to a nucleotide sequence coding for a polypeptide.

The term "operably linked" as used herein is to be understood as that the promoter or promoter variant is linked with the nucleotide sequence coding for a polypeptide in an orientation that allows facilitation of gene expression, i.e. transcription, of the nucleotide sequence coding for a polypeptide by the promoter or promoter variant. In other words, the 3'-end of the promoter or promoter variant is linked to the 5'-end of the nucleotide sequence comprising an open reading frame coding for a polypeptide. The 5'-end of the open reading frame coding for a polypeptide is typically a start codon that encodes the first amino acid of the polypeptide. Often, although not necessarily always, this start codon has the sequence 5'-A-T-G-3', wherein the 5'-end of the open reading frame coding for a polypeptide is "A", i.e. the nucleotide deoxyadenosine monophosphate, and which start codon typically encodes the amino acid methionine. Typically, the 5'-end of the open reading frame coding for a polypeptide is positioned in proximity to the 3'-end of the promoter or promoter variant. The 5'-end of the open reading frame coding for a polypeptide may be linked directly to the 3'-end of the promoter or promoter variant. Alternatively, the 5'-end of the open reading frame coding for a polypeptide may be separated from the 3'-end of the promoter or promoter variant by one or more nucleotides without substantially altering the activity of the promoter or promoter variant on the expression of the open reading frame coding for a polypeptide operably linked to the promoter or promoter variant. It is considered that the one or more nucleotides separating the 3'-end of the promoter or promoter variant from the 5'-end of the open reading frame coding for a polypeptide may be e.g. a nucleotide sequence which is recognized by one or more restriction enzymes. Preferably, the one or more nucleotides separating the 3'-end of the promoter or promoter variant from the 5'-end of the open reading frame coding for a polypeptide is shorter than 100 nucleotides, more preferably shorter than 50 nucleotides, even more preferably shorter than 20 nucleotides, most preferably shorter than 10 nucleotides. The terms "protein" and "polypeptide" are used interchangeably.

The present invention further relates to a promoter or promoter variant as described herein, wherein the promoter or promoter variant is comprised in a yeast cell, and wherein the promoter or promoter variant is operably linked to a nucleotide sequence coding for a polypeptide. Hereby, biotechnological protein production can be achieved in a suitable host organism. In a preferred embodiment, the yeast cell is a cell of a genus selected from the group consisting of *Pichia, Komagataella, Ogataea, Candida, Hansenula, Saccharomyces, Schizosaccharomyces, Kluyveromyces*, *Zygosaccharomyces* and *Yarrowia,* preferably a *Pichia pastoris* cell or a *Yarrowia lipolytica* cell, most preferably a *Pichia pastoris* cell. By selecting the yeast cell from the genus group consisting of *Pichia*, *Komagataella*, *Ogataea*, *Candida*, *Hansenula*, *Saccharomyces*, *Schizosaccharomyces*, *Kluyveromyces*, *Zygosaccharomyces* and *Yarrowia,* and in particular by selecting the yeast cell from the organisms *Pichia pastoris* or *Yarrowia lipolytica*, preferably *Pichia pastoris,* protein production can be achieved in a particularly efficient manner. A person skilled in the art is aware of the fact that the name "*Pichia pastoris*", as used herein, refers to several yeast strains that have also been classified to the taxa of *Komagataella pastoris* and *Komagataella phaffii* (Yamada et al. Biosci Biotechnol Biochem. 1995. 59(3): 439-444; Kurtzman. J Ind Microbiol Biotechnol. 2009. 35(11): 1435-1438). Further synonymous, albeit less common names for *P*. *pastoris* strains are *Zygosaccharomyces pastoris* Guilliermond, *Zygosaccharomyces pastori* Guilliermond, *Endomyces pastoris* (Guilliermond), *Endomyces pastori* (Guilliermond), *Saccharomyces pastoris* (Guillliermond), *Saccharomyces pastori* (Guillliermond), *Petasospora pastori* (Guilliermond), *Zymopichia pastoris* (Guilliermond), *Zymopichia pastori* (Guilliermond) or *Zygowilia pastori* (Guilliermond) (Wolf et al. Springer-Verlag Berlin Heidelberg. 2003. Non-concentional yeasts in genetics, biochemistry and biotechnology. ISBN 978-3-540-44215-8: p. 489; http://www.mycobank.org/BioloMICS.aspx?TableKey=14682616000000067&Rec=107579&Fiel ds=All). *Pichia pastoris* platform strains that are of particular industrial relevance are *inter alia Komagataella phaffii* CBS 7435 (also deposited e.g. as ATCC 76273 or NRRL Y-11430), or *Komagataella phaffii* GS115 (also deposited e.g. as ATCC 20864), or *Komagataella pastoris* CBS 704 (also deposited e.g. as ATCC 28485 or NRRL Y-1603).

It is another objective of the present invention to provide a method that allows high product titers by recombinant protein production. Therefore, another aspect of the present invention relates to a method for recombinant production of a target polypeptide comprising the steps of introducing an expression cassette comprising a nucleotide sequence encoding the target polypeptide into at least one yeast cell; producing a yeast cell culture by multiplying the at least one yeast cell; subjecting the yeast cell culture to a condition suitable for expression of the nucleotide sequence encoding the target polypeptide; and isolating the produced target protein from the yeast cell culture; wherein the expression cassette further comprises a promoter or promoter variant as described herein operably linked to the nucleotide sequence encoding the target polypeptide. By applying such a method, it was surprisingly found that especially high amounts of target polypeptide could be produced. It is noted that the method for recombinant production of a target polypeptide according to the present invention may also be performed in a manner, wherein the expression cassette comprises a promoter comprising the nucleotide sequence of SEQ ID NO: 1.

An expression cassette can be introduced into a yeast cell by applying common yeast transformation protocols known to a person having skill in the art, e.g. by electroporation (Lin-Cereghino et al. Biotechniques. 2005. 38(1): 44-48). An expression cassette may be provided by itself but also as part of larger polynucleotide constructs such as linear or circular plasmids. A yeast cell culture can be produced by multiplication of one or more yeast cell(s) by applying cultivation protocols suitable for cultivating yeast as known in the state-of-the-art, e.g. Krainer et al. (Microb Cell Fact. 2012. 11:22) or Cregg (Humana Press Inc. 2007. Pichia Protocols. ISBN 978-1-59745-456-8). To isolate a produced target protein from a yeast cell culture several options are available, including but not limited to the separation of the cells from the liquid by centrifugation and collecting the supernatant comprising a secreted target protein, or lysing the yeast cells and collecting the lysate comprising produced target protein. In addition, further purification steps such as e.g. fractionated precipitation or chromatographic methods can be applied to isolate produced target protein.

In a preferred embodiment of the invention, the method as described herein is performed in a manner, wherein the condition suitable for expression of the target polypeptide is a fed-batch process. Preferably, the fed-batch process is operated with a glucose feed rate of at least 0.04 mmol of glucose per gram of cell dry weight and per hour (mmol Glu/g CDW/h) or a glycerol feed rate of at least 0.07 mmol of glycerol per gram of cell dry weight and per hour (mmol Gly/g CDW/h), preferably with a glucose feed rate of at least 0.11 mmol Glu/g CDW/h or a glycerol feed rate of at least 0.21 mmol Gly/g CDW/h, more preferably with a glucose feed rate of at least 0.18 mmol Glu/g CDW/h or a glycerol feed rate of at least 0.35 mmol Gly/g CDW/h. In contrast to the current teaching in the state-of-the-art, not derepression by depletion of glucose or glycerol, but active feeding with either glucose or glycerol as described herein was found to increase the production of a target polypeptide under control of a HpFMD-based promoter, in particular when a glucose feed rate of at least 0.04 mmol Glu/g CDW/h or a glycerol feed rate of at least 0.07 mmol Gly/g CDW/h, preferably at least 0.11 mmol Glu/g CDW/h or at least 0.21 mmol Gly/g CDW/h, more preferably at least 0.18 mmol Glu/g CDW/h or at least 0.35 mmol Gly/g CDW/h, is applied. In a preferred embodiment of the invention, the yeast cell is a *Pichia pastoris* cell or a *Yarrowia lipolytica* cell, preferably a *Pichia pastoris* cell. Hereby, recombinant production by a method as described herein can be performed to yield especially high product titers.

Another aspect of the present invention relates to a method for increasing the relative expression efficiency of a nucleotide sequence having promoter activity, the method comprising the step of providing a truncated promoter by truncating a nucleotide sequence having at least 80%, preferably at least 90% identity, more preferably at least 95% identity, more preferably at least 97% identity, more preferably at least 98% identity, even more preferably at least 99% identity, most preferably 100% identity to the nucleotide sequence of SEQ ID NO: 1 from the 5'-end to a length of 140-610 base pairs, preferably to a length of 140-560 base pairs, more preferably to a length of 160-560 base pairs, most preferably to a length of 160-500 base pairs. Merely for the avoidance of doubt, the truncation is performed solely from the 5'-end while the 3'-end of the nucleotide sequence remains untruncated. Preferably, the relative expression efficiency of the truncated promoter is at least 5% higher, preferably at least 10% higher, more preferably at least 20% higher, even more preferably at least 50% higher, most preferably at least 100% higher than the relative expression efficiency of the promoter having the nucleotide sequence of SEQ ID NO: 1.

The present invention is further characterized by the following items:
1. Promoter, wherein the promoter is truncated from the 5'-end of the nucleotide sequence of SEQ ID NO: 1 to a length of 160-500 base pairs; or a variant thereof (promoter variant), wherein the promoter variant has at least 80% identity, preferably at least 90% identity, more preferably at least 95% identity, even more preferably at least 96% identity, even more preferably at least 97% identity, even more preferably at least 98%, most preferably at least 99% identity to the promoter.
2. Promoter according to item 1, wherein the promoter or the promotor variant contains at least one non-naturally occurring substitution modification relative to SEQ ID NO: 1.
3. Promoter, wherein the promoter is truncated from the 5'-end of any one of the nucleotide sequences of SEQ ID NOs: 1, 6-24 to a length of 560 base pairs, preferably 500 base pairs, more preferably 450 base pairs, more preferably 380 base pairs, more preferably 300 base pairs, more preferably 280 base pairs, more preferably 170 base pairs, most preferably 160 base pairs.
4. Promoter, wherein the promoter is truncated from the 5'-end of the nucleotide sequence of SEQ ID NO: 1 to a length of 560 base pairs, preferably 500 base pairs, more preferably 450 base pairs, more preferably 380 base pairs, more preferably 300 base pairs, more preferably 280 base pairs, more preferably 170 base pairs, most preferably 160 base pairs.
5. Promoter according to item 4, wherein the promoter is truncated from the 5'-end of the nucleotide sequence of SEQ ID NO: 1 to a length of 160 base pairs, and wherein the promoter has the nucleotide sequence of SEQ ID NO: 5.
6. Promoter or promoter variant according to any one of the items 1 to 5, wherein the relative expression efficiency of the promoter or promoter variant is at least 5%, preferably at least 10%, more preferably at least 30%, more preferably at least 40%, more preferably at least 50%, most preferably at least 100% higher than the relative expression efficiency of the promoter having the nucleotide sequence of SEQ ID NO: 1.
7. Expression cassette comprising a promoter or promoter variant according to any one of the items 1 to 6, and a nucleotide sequence coding for a polypeptide and optionally a transcription terminator sequence, wherein the promoter or promoter variant is operably linked to the nucleotide sequence coding for a polypeptide.
8. Expression cassette according to item 7, wherein the nucleotide sequence coding for a polypeptide is a heterologous nucleic acid sequence.
9. Expression cassette according to any one of the items 7 and 8, wherein the nucleotide sequence coding for a polypeptide codes for a heterologous polypeptide.
10. Plasmid comprising a promoter or promoter variant according to any one of the items 1 to 6, or an expression cassette according to any one of the items 7 to 9.
11. Yeast cell comprising a promoter or promoter variant according to any one of the items 1 to 6, or an expression cassette according to any one of the items 7 to 9, or a plasmid according to item 10, wherein the yeast cell is a cell of a genus selected from the group consisting of *Pichia*, *Komagataella*, *Ogataea*, *Candida*, *Hansenula*, *Saccharomyces*, *Schizosaccharomyces*, *Kluyveromyces*, *Zygosaccharomyces* and *Yarrowia*, preferably a *Pichia pastoris* cell or a *Yarrowia lipolytica* cell, most preferably a *Pichia pastoris* cell.
12. Method for recombinant production of a target polypeptide comprising the steps of a) introducing an expression cassette comprising a nucleotide sequence encoding the target polypeptide into at least one yeast cell; b) producing a yeast cell culture by multiplying the at least one yeast cell; c) subjecting the yeast cell culture to a condition suitable for expression of the nucleotide sequence encoding the target polypeptide; and d) isolating the produced target protein from the yeast cell culture, wherein the expression cassette comprises a promoter or promoter variant according to any one of the items 1 to 6 operably linked to the nucleotide sequence encoding the target polypeptide.
13. Method for recombinant production of a target polypeptide comprising the steps of a) introducing an expression cassette comprising a nucleotide sequence encoding the target polypeptide into at least one yeast cell; b) producing a yeast cell culture by multiplying the at least one yeast cell; c) subjecting the yeast cell culture to a condition suitable for expression of the nucleotide sequence encoding the target polypeptide; and d) isolating the produced target protein from the yeast cell culture, wherein the expression cassette comprises a promoter comprising the nucleotide sequence of SEQ ID NO: 1 operably linked to the nucleotide sequence encoding the target polypeptide.
14. Method according to any one of the items 12 and 13, wherein the expression cassette further comprises a transcription terminator sequence.
15. Method according to any one of the items 12 to 14, wherein the condition suitable for expression of the nucleotide sequence encoding the target polypeptide is a fed-batch process.
16. Method according to item 15, wherein the fed-batch process is operated with a glucose feed rate of at least 0.04 mmol of glucose per gram of cell dry weight and per hour (mmol Glu/g CDW/h) or a glycerol feed rate of at least 0.07 mmol of glycerol per gram of cell dry weight and per hour (mmol Gly/g CDW/h), preferably with a glucose feed rate of at least 0.11 mmol Glu/g CDW/h or a glycerol feed rate of at least 0.21 mmol Gly/g CDW/h, more preferably with a glucose feed rate of at least 0.18 mmol Glu/g CDW/h or a glycerol feed rate of at least 0.35 mmol Gly/g CDW/h.
17. Method according to any one of the items 12 to 16, wherein the nucleotide sequence encoding the target polypeptide is a heterologous nucleic acid sequence.
18. Method according to item 17, wherein the nucleotide sequence encoding the target polypetpide codes for a heterologous polypeptide.
19. Method according to any one of the items 12 to 18, wherein the yeast cell is a *Pichia pastoris* cell or a *Yarrowia lipolytica* cell, preferably a *Pichia pastoris* cell.
20. Method for increasing the relative expression efficiency of a nucleotide sequence having promoter activity, the method comprising the step of providing a truncated promoter by truncating a nucleotide sequence having at least 80%, preferably at least 90% identity, more preferably at least 95% identity, more preferably at least 97% identity, more preferably at least 98% identity, even more preferably at least 99% identity, most preferably 100% identity to the nucleotide sequence of SEQ ID NO: 1 from the 5'-end to a length of 140-610 base pairs, preferably to a length of 140-560 base pairs, more preferably to a length of 160-560 base pairs, most preferably to a length of 160-500 base pairs.
21. Method according to item 20, wherein the promoter or the promotor variant contains at least one non-naturally occurring substitution modification relative to SEQ ID NO: 1.
22. Method according to any one of the items 20 and 21, wherein the relative expression efficiency of the truncated promoter is at least 5%, preferably at least 10%, more preferably at least 20%, even more preferably at least 50%, most preferably at least 100% higher than the relative expression efficiency of the promoter having the nucleotide sequence of SEQ ID NO: 1.
23. Method according to any one of the items 20 to 22, wherein the relative expression efficiency of the truncated promoter is increased in a yeast cell, wherein the yeast cell is a *Pichia pastoris* cell or a *Yarrowia lipolytica* cell, preferably a *Pichia pastoris* cell.

In the following, the solution of the present invention is further described by non-limiting figures and examples.
Figure 1 is a bar chart showing the signals obtained from a reporter protein produced under the control of a promoter having the nucleotide sequence of SEQ ID NO: 1 and of truncated promoter variants thereof.
Figure 2 is a bar chart showing the signals obtained from a reporter protein produced under the control of a promoter having the nucleotide sequence of SEQ ID NO: 2 and of truncated promoter variants thereof as comparative example to the signals obtained from a reporter protein produced under the control of a promoter having the nucleotide sequence of SEQ ID NO: 1 and truncated promoter variants thereof.
Figure 3 is a bar chart showing the signals obtained from a reporter protein produced under the control of a promoter having the nucleotide sequence of SEQ ID NO: 3 and of truncated promoter variants thereof as comparative example to the signals obtained from a reporter protein produced under the control of a promoter having the nucleotide sequence of SEQ ID NO: 1 and truncated promoter variants thereof.
Figure 4 is a bar chart showing the signals obtained from a reporter protein produced under the control of a promoter having the nucleotide sequence of SEQ ID NO: 4 and of truncated promoter variants thereof as comparative example to the signals obtained from a reporter protein produced under the control of a promoter having the nucleotide sequence of SEQ ID NO: 1 and truncated promoter variants thereof.
Figure 5 is a bar chart showing the amount of volumetric enzyme activity as reporter signal obtained from a phosphatase enzyme (UniProtKB entry: P11491) recombinantly produced under the control of a promoter having the nucleotide sequence of SEQ ID NO: 1 and of truncated promoter variants thereof.
Figure 6 is a bar chart showing the amount of volumetric enzyme activity as reporter signal obtained from an alcohol dehydrogenase enzyme recombinantly produced under the control of a promoter having the nucleotide sequence of SEQ ID NO: 1 and of truncated promoter variants thereof.
Figure 7 is a bar chart showing the amount of volumetric enzyme activity as reporter signal obtained from a peroxidase enzyme (UniProtKB entry: P00433) recombinantly produced under the control of a promoter having the nucleotide sequence of SEQ ID NO: 1 and of truncated promoter variants thereof.
Figure 8 is a graph chart showing the amount of volumetric enzyme activity as reporter signal produced under the control of a promoter having the nucleotide sequence of SEQ ID NO: 1 throughout a cultivation.
Figure 9 is a graph chart showing the amount of volumetric enzyme activity as reporter signal produced under the control of a truncated variant of the promoter having the nucleotide sequence of SEQ ID NO: 1 throughout a cultivation.
Figure 10 is a graph chart showing the amounts of volumetric enzyme activity as reporter signals produced by several strains, wherein the reporter was produced under the control of a truncated variant of the promoter having the nucleotide sequence of SEQ ID NO: 1 throughout a cultivation.

### Detailed description

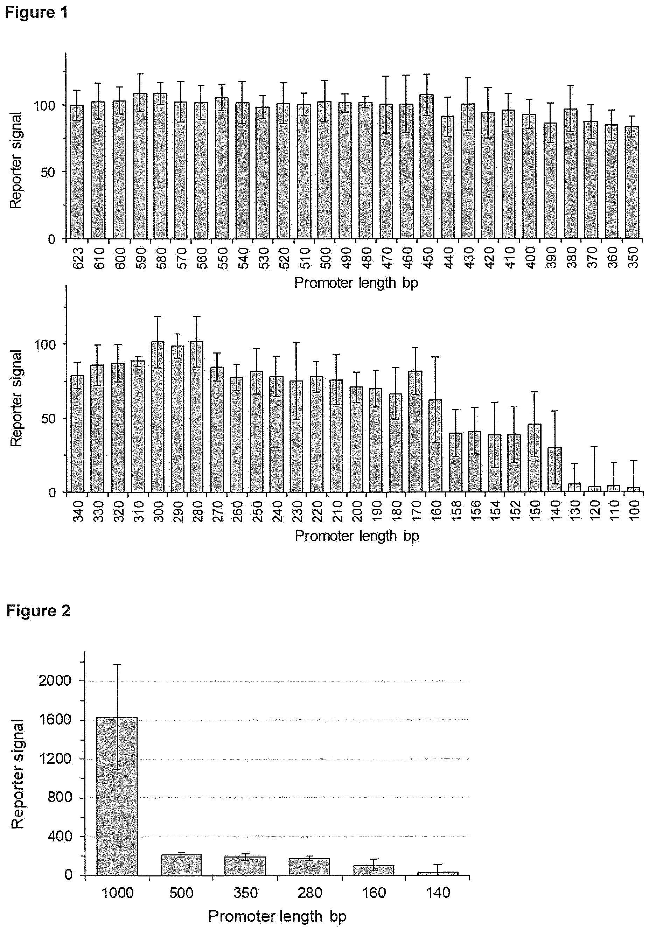

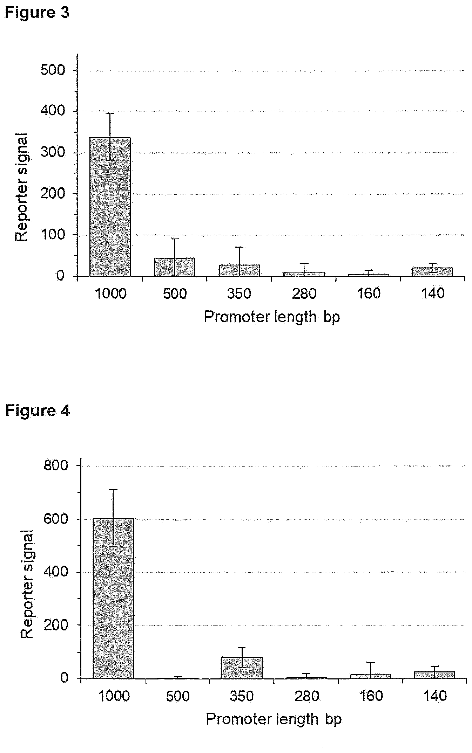

Referring to Figs. 1-4, bar charts of the signals obtained from reporter protein produced under the control of a promoter having either of the nucleotide sequences of SEQ ID NOs: 1-4 and of truncated variants thereof are shown. Shown is the median value with standard deviations obtained from at least nine different transformant strains per promoter construct. While the promoter having the nucleotide sequence of SEQ ID NO: 1 was surprisingly found to tolerate a remarkable degree of truncation to a length as short as 140 bp (Fig. 1), neither the promoter of the *P*. *pastoris ADH2* gene encoding the alcohol dehydrogenase 2 enzyme of *P*. *pastoris* (SEQ ID NO: 2; Fig. 2), nor the promoter of the *P*. *pastoris AOX1* gene encoding the alcohol oxidase 1 enzyme of *P*. *pastoris* (SEQ ID NO: 3; Fig. 3), nor the promoter of the *P*. *pastoris FDH1* gene encoding the formate dehydrogenase 1 enzyme of *P*. *pastoris* (SEQ ID NO: 4; Fig. 4) allowed this approach without a substantial loss of promoter activity. It thus appears that this tolerance for truncation of the promoter having the nucleotide sequence of SEQ ID NO: 1 is the exception rather than the rule.

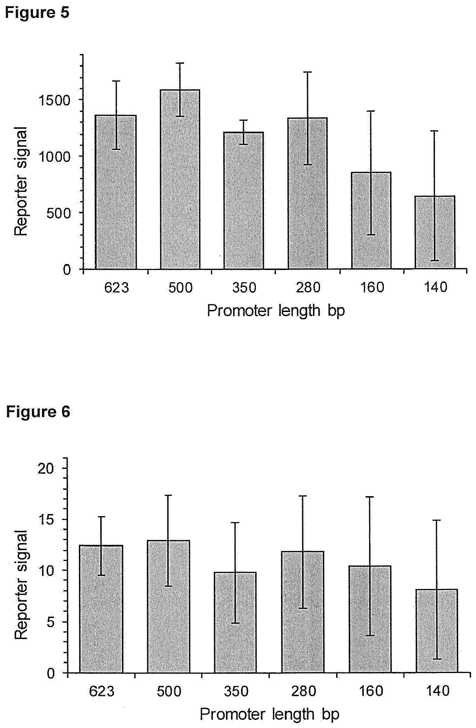

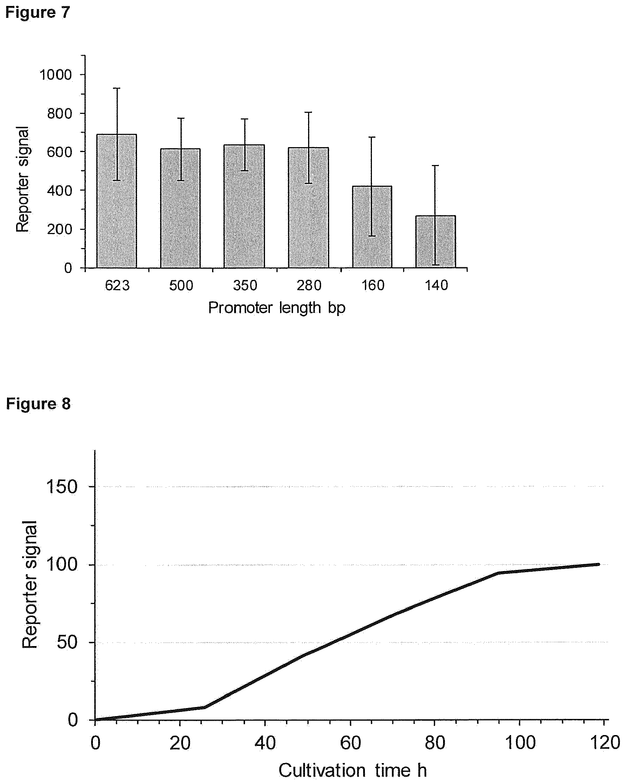

Referring to Figs. 5-7, bar charts of the amounts of volumetric enzyme activity as reporter signal from three different reporter enzymes are shown, which were produced under the control of a promoter having the nucleotide sequence of SEQ ID NO: 1 and of truncated variants thereof. Shown are bars representing average values with standard deviations obtained from at least nine different transformant strains upon cultivation in 96-deep well plates. Essentially the same effect was seen for a phosphatase enzyme (Fig. 5), an alcohol dehydrogenase enzyme (Fig. 6), and a peroxidase enzyme (Fig. 7) as for an esterase enzyme (Fig. 1). Based on these results it is concluded that expression from truncated variants of a promoter having the nucleotide sequence of SEQ ID NO: 1 occurs independently from the protein encoded in the open reading frame whose expression is regulated by the promoter.

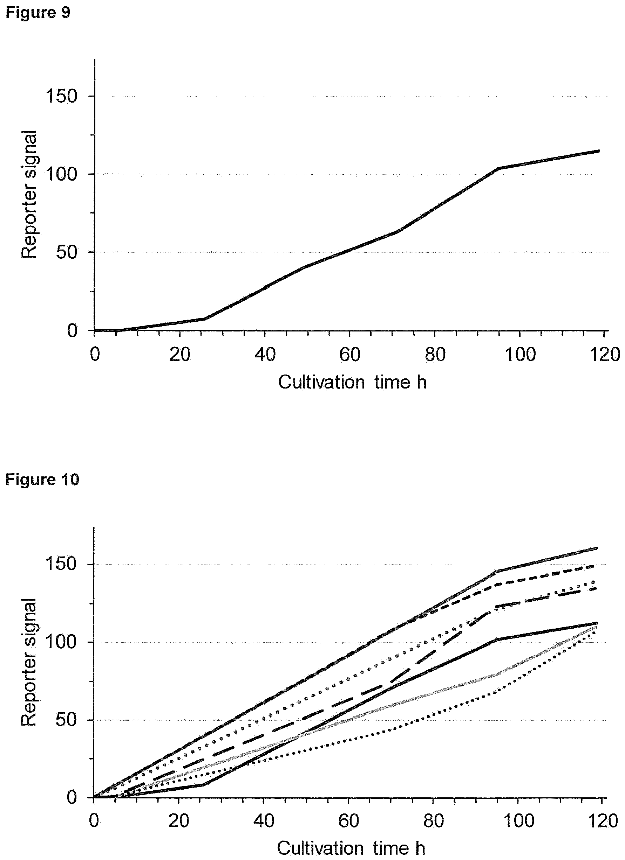

Referring to Figs. 8-10, graph charts of volumetric enzyme activities as reporter signals are shown that were produced throughout bioreactor cultivations either under the control of a promoter having the nucleotide sequence of SEQ ID NO: 1 (Fig. 8), or under the control of truncated variants thereof (Figs. 9 and 10). A strain harboring an expression cassette comprising a 280 bp long, truncated promoter of the promoter having the nucleotide sequence of SEQ ID NO: 1 (Fig. 9), as well as several different strains harboring an expression cassette comprising a 160 bp long, truncated promoter of the promoter having the nucleotide sequence of SEQ ID NO: 1 (Fig. 10) were found to produce at least the same amount of target enzyme as a strain harboring an expression cassette comprising the 623 bp long, non-truncated promoter having the nucleotide sequence of SEQ ID NO: 1.

### Examples

### Example 1: Expression studies with truncated eukaryotic promoters in Pichia pastoris

To assess whether eukaryotic promoters can be truncated without a substantial loss of promoter activity, full length polynucleotide fragments of selected promoters were either ordered as synthetically produced fragments or amplified directly from the respective eukaryotic organisms. In particular, the *Ogataea polymorpha FMD* promoter (SEQ ID NO: 1), the *Pichia pastoris ADH2* promoter (SEQ ID NO: 2), the *P. pastoris AOX1* promoter (SEQ ID NO: 3), and the *P*. *pastoris FDH1* promoter (SEQ ID NO: 4) were studied. To obtain truncated variants of these promoters, oligonucleotide primers were designed for amplification via polymerase chain reaction (PCR), wherein a reverse primer was designed to bind to the 3' end of the promoter, and forward primers were designed to bind within the full length promoter sequence depending on the desired final length of the PCR product. For the *Ogataea polymorpha FMD* promoter, truncated promoters having either 612, 610, 600, 590, 580, 570, 560, 550, 540, 530, 520, 510, 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, 190, 180, 170, 160, 158, 156, 154, 152, 150, 150, 130, 120, 110, 100 bp were tested. For the *P*. *pastoris ADH2* promoter, the *P*. *pastoris AOX1* promoter, and the *P*. *pastoris FDH1* promoter, variants having either 1000, 500, 350, 280, 160, 140 bp were tested. The full length promoters or the truncated variants thereof were cloned into expression cassettes for recombinant gene expression in *P*. *pastoris,* using state-of-the-art standard cloning methods (e.g. J.M. Cregg, Pichia Protocols, second Edition, ISBN-10: 1588294293, 2007; J. Sambrook et al. 2012. Molecular Cloning, A Laboratory Manual 4th Edition, Cold Spring Harbor). These expression cassettes comprised either the full length promoters or the truncated variants thereof, an open reading frame encoding a secretory reporter protein, a transcription terminator (the *P. pastoris AOX1* transcription terminator), and a cassette for an antibiotic selection marker. To quantify the amount of produced target protein by the respective full length promoters or the truncated variants thereof, an esterase enzyme (UniProtKB entry: D2D3B6) was used as reporter protein. An N-terminal fusion of the prepro peptide of the *Saccharomyces cerevisiae* mating factor alpha to the esterase was used to achieve secretion of the esterase by transformant cells, which prepro peptide replaced the initial 47 amino acids of the full length esterase polypeptide.

Transformation of a *P*. *pastoris* wildtype strain with either of the generated expression cassettes was achieved via electroporation, essentially following the protocol of Lin-Cereghino et al. (Biotechniques. 2005. 38(1): 44-48). However, any alternative protocol for transformation of *P*. *pastoris* would also be suitable. After transformation, the cells were incubated on agar plates containing the appropriate antibiotic for selection of transformant strains.

Microscale cultivations of at least nine transformant strains per transformed expression cassette were performed in 96-deep well plates (Hamilton 2.2mL PP deep well plates) similar to the protocol described by Weis et al. (FEMS Yeast Res. 2004. 5(2): 179-189): Cells were grown in 450 µL of YPD (1% w/v yeast extract, 2% w/v peptone, 2% w/v glucose) per well at 28 °C, 320 rpm, 90% humidity in a Kuhner ISF1-X Climo-Shaker (throw 50 mm) for approximately 60 hours. In the case of strains transformed with expression cassettes comprising either the *AOX1* promoter or the *FDH1* promoter, expression was induced by addition of 100 µL of YPM0.5% (1% w/v yeast extract, 2% w/v peptone, 0.5% w/v methanol) per well, 60 h after inoculation. In the case of strains transformed with expression cassettes comprising either the *FMD* promoter or the *ADH1* promoter, 100 µL of YPD per well was added, 60 h after inoculation. After approximately 120 hours of cultivation, the 96-deep well plates were subjected to centrifugation to separate the biomass from the supernatant. The supernatant was used for analysis of produced secreted target reporter protein. To allow plate-to-plate comparability, a stable esterase producing strain was also cultivated in at least four wells of each plate as positive control and calibrator strain.

The amount of target reporter protein secreted into the supernatant was determined by quantifying the amount of volumetric esterase activity in the supernatant: Enzymatic activity was quantified by determining the hydrolysis of the esterase substrate 4-nitrophenyl 2-(trimethylsilyl)ethyl carbonate, which was determined photometrically by following the increase in absorbance at 405 nm as described by Ruth et al. (Microb Cell Fact. 2014. 13: 120). The amounts of signal of produced reporter protein by the tested expression cassettes are shown in Figures 1, 2, 3 and 4 for the non-truncated promoters of *FMD*, *ADH2*, *AOX1* and *FDH1,* respectively, and for truncated promoter variants thereof. It is well known in the art that *P*. *pastoris* transformants tend to show clonal variation (e.g. Vogl et al. Appl Environ Microbiol. 2018. 84(6): e02712-17). In contrast to other expression systems such as *Escherichia coli*, it is thus necessary to test not only one transformant strain, but several strains - as described herein. It is worth noting that clonal variation was found more pronounced when a promoter was shorter, as reflected in higher standard deviations and as seen e.g. for truncated variants of the *FMD* promoter having 160 bp or 150 bp compared to longer variants having 600 bp. Without wishing to be bound by theory, this effect is believed to be due to a larger impact of the site of integration of a shorter promoter.

The median of produced enzyme activity by at least nine transformant strains per expression cassette was used to calculate the relative expression efficiency of the respective promoter or promoter variant. The relative expression efficiency was calculated by dividing the median of produced enzyme activity by the length of the promoter used to control the expression of the target reporter gene. In the case of the *FMD* promoter, truncated promoter variants having a length between 140 and 610 bp were found to have at least a 5% increase in relative expression efficiency compared to the full length promoter, see Table 1 below. Essentially the same results were obtained irrespective which *P. pastoris* strain (*K. phaffii* CBS 7435, *K*. *phaffii* GS115 or *K*. *pastoris* CBS 704) was transformed.

**Table 1: Relative expression efficiencies (REE) of the full length FMD promoter (623 bp) and of truncated variants thereof.**

| **Length bp** | **REE %** | **Length bp** | **REE %** | **Length bp** | **REE %** | **Length bp** | **REE %** |
|---|---|---|---|---|---|---|---|
| 623 | 100.0 | 470 | 133.5 | 320 | 169.9 | 170 | 300,5 |
| 610 | 105.3 | 460 | 136.8 | 310 | 178.5 | 160 | 242,6 |
| 600 | 107.7 | 450 | 149.5 | 300 | 211.6 | 158 | 156,7 |
| 590 | 115.8 | 440 | 129.5 | 290 | 212.4 | 156 | 164,5 |
| 580 | 117.5 | 430 | 146.2 | 280 | 226.7 | 154 | 155,1 |
| 570 | 112.5 | 420 | 140.0 | 270 | 195.3 | 152 | 157,7 |
| 560 | 114.0 | 410 | 146.2 | 260 | 185.8 | 150 | 189,5 |
| 550 | 120.5 | 400 | 145.2 | 250 | 204.2 | 140 | 133,1 |
| 540 | 117.7 | 390 | 138.3 | 240 | 203.0 | 130 | 26,3 |
| 530 | 116.2 | 380 | 159.2 | 230 | 203.8 | 120 | 17,5 |
| 520 | 122.0 | 370 | 147.4 | 220 | 221.6 | 110 | 22,5 |
| 510 | 123.2 | 360 | 147.1 | 210 | 225.7 | 100 | 18,6 |
| 500 | 128.5 | 350 | 148.8 | 200 | 220.8 | | |
| 490 | 129.7 | 340 | 144.6 | 190 | 229.7 | | |
| 480 | 133.0 | 330 | 162.5 | 180 | 230.8 | | |

To demonstrate the mutational stability of truncated promoter variants, mutant variants having at least 80% sequence identity to a promoter truncated to a length of 160 bp from the 5'-end of the nucleotide sequence of SEQ ID NO: 1 were tested. The mutant variants were truncated to a length of 160 bp from the 5'-end of any one of the nucleotide sequences of SEQ ID NOs: 6-24, whereby these truncated mutant variants have sequence identities from 80.74% to 99.38% to the promoter truncated to a length of 160 bp from the 5'-end of the nucleotide sequence of SEQ ID NO: 1. For all mutant variants, the same effect was observed as for truncated variants of the promoter of the nucleotide sequence of SEQ ID NO: 1. Despite an almost 4-fold reduction in length, reporter signals were obtained comparable to the non-truncated promoter, thus achieving at least an increase of 5% in relative expression efficiency of the new truncated promoters.

In contrast, none of the truncated variants of either of the promoters of *ADH2*, *AOX1* or *FDH1* resulted in an increased relative expression efficiency compared to the respective full length promoters (each 1000 bp in length), see Table 2 below.

**Table 2: Relative expression efficiencies (REE) of the full length promoters of either ADH2, AOX1 or FDH1 (each 1000 bp) and of truncated variants thereof.**

| ***ADH2* promoter** | | ***AOX1* promoter** | | ***FDH1* promoter** | |
|---|---|---|---|---|---|
| **Length bp** | **REE %** | **Length bp** | **REE %** | **Length bp** | **REE %** |
| 1000 | 100.0 | 1000 | 100.0 | 1000 | 100.0 |
| 500 | 26.7 | 500 | 27.1 | 500 | 0.5 |
| 350 | 33.9 | 350 | 24.2 | 350 | 38.9 |
| 280 | 38.9 | 280 | 9.2 | 280 | 4.0 |
| 160 | 40.8 | 160 | 9.2 | 160 | 18.4 |
| 140 | 12.0 | 140 | 42.7 | 140 | 29.6 |

In conclusion, it was surprisingly found that only certain truncated variants of the *O*. *polymorpha FMD* promoter, but not truncated variants of any of the other tested eukaryotic promoters resulted in an increased relative expression efficiency by *P. pastoris* when compared to a non-truncated full length promoter.

### Example 2: Recombinant production of different reporter proteins using promoters having increased relative expression efficiency in small-scale cultivations

Expression regulation by a promoter occurs on DNA level, in particular on the level of the DNA upstream the open reading frame whose expression is regulated by said promoter. Therefore, a person skilled in the art would not expect substantial differences in promoter activity when the expression of different open reading frames is regulated. In other words, production of proteins different from the esterase described in Example 1 would not be expected to yield substantially different results with regards to the promoter activity or functionality than production of the esterase as described in Example 1. To nevertheless experimentally verify a general applicability of the truncated promoter variants having increased relative expression efficiency for the expression of any recombinant target gene, further expression cassettes were generated harboring open reading frames encoding either a phosphatase enzyme, an alcohol dehydrogenase enzyme or a peroxidase enzyme.

Expression of the genes comprising an open reading frame encoding either a phosphatase enzyme, an alcohol dehydrogenase enzyme or a peroxidase enzyme was designed to be regulated by either the full length *O*. *polymorpha FMD* promoter (623 bp), or by truncated variants thereof having a length of either 500 bp, 350 bp, 280 bp, 160 bp or 140 bp. Transformations of *P*. *pastoris* and cultivations in 96-deep well plates were performed essentially as described for this promoter in Example 1.

To determine the amounts of produced phosphatase, a para-nitrophenol (pNPP) assay was performed essentially as described in Takai and Mieskes (Biochem J. 1991. 275(1): 233-239), wherein assay solutions contained 10 mM pNPP, 1 mM MgCl₂, 0.1 mM ZnCl₂, 40 mM Tris-HCI, pH 8.1. To determine the amounts of produced alcohol dehydrogenase, the enzymatic activity was determined essentially as described in WO2016154640A1. *P. pastoris* strains producing alcohol dehydrogenase were cultivated in YPD comprising additional 1 mM pyrroloquinoline quinone and 1 mM calcium chloride. To determine the amounts of produced alcohol dehydrogenase, the enzymatic activity was determined by mixing 5 µL of clear culture supernatant comprising the produced enzyme with 280 µL of assay buffer (20 mM potassium-hexacyanoferrate (III), 50 mM Tris-HCI, pH 7.5, 50 ppm deoxynivalenol). Assay reactions were incubated for 60 min at 30 °C. Fifty µL samples were drawn regularly from the reactions and analyzed via HPLC. To determine the amounts of produced peroxidase, the enzymatic activity was measured analogous to Krainer et al. (J Biotechnol. 201. 233:181-189), exchanging the substrate ABTS (CAS No.: 30931-67-0) with TMB (3,3',5,5'-tetramethybenzidine; CAS No.: 54827-17-7); product formation was followed at 653 nm. The amounts of produced reporter enzyme signal by the tested promoter variants are shown in Figures 5, 6 and 7 for phosphatase, alcohol dehydrogenase and peroxidase, respectively.

Relative expression efficiencies were determined analogous to the protocol described in Example 1, and are shown in Table 3.

**Table 3: Relative expression efficiencies (REE) of produced phosphatase, alcohol dehydrogenase or peroxidase under control of either the full length promoter of FMD (623 bp) or truncated variants thereof (500 bp, 350 bp, 280 bp, 160 bp, 140 bp).**

| **Phosphatase** | | **Alcohol dehydrogenase** | | **Peroxidase** | |
|---|---|---|---|---|---|
| **Length bp** | **REE %** | **Length bp** | **REE %** | **Length bp** | **REE %** |
| 623 | 100.0 | 623 | 100.0 | 623 | 100.0 |
| 500 | 145.0 | 500 | 130.1 | 500 | 110.9 |
| 350 | 158.7 | 350 | 140.5 | 350 | 164.3 |
| 280 | 217.8 | 280 | 212.0 | 280 | 200.4 |
| 160 | 242.5 | 160 | 326.7 | 160 | 237.5 |
| 140 | 210.9 | 140 | 289.8 | 140 | 173.7 |

In conclusion, it was found that the functionality and applicability of the truncated promoter variants of the *FMD* wildtype promoter are not limited to a single open reading frame, but well-suited to regulate expression of any target gene.

### Example 3: Recombinant protein production using promoters having increased relative expression efficiency in bioreactor cultivations

It is known that microbial small-scale cultivations performed e.g. in 24- or 96-well plates or in shake flasks tend to yield different data than cultivations performed in bioreactors. Without wishing to be bound by theory it is believed that these differences stem from the different conditions with regards to nutrient supply as well as to pH and temperature control, oxygen supply and agitation among the types of cultivation. While small-scale cultivations are well suited e.g. to test the general concept of scientific hypotheses and to screen large numbers of microbial transformants for production strains, bioreactor cultivations are the preferred choice to evaluate production performance data.

To demonstrate the applicability of truncated variants of the *O*. *polymorpha FMD* promoter not only in small-scale cultivations but also in large-scale cultivations, *Pichia pastoris* strains harboring expression cassettes comprising truncated *FMD* promoter variants were cultivated in bioreactors, in comparison to a strain harboring an expression cassette comprising the non-truncated *FMD* promoter as benchmark. As reporter enzyme, the esterase enzyme of Example 1 was used.

Precultures were grown in YPG media containing 10 g/kg yeast extract, 20 g/kg soy peptone, 0.3 mL/kg antifoam Struktol J650 and 40 g/kg glycerol in 500 mL baffled shake flasks at 180 rpm and at 28°C for 24 h. Five hundred mL of batch media as reported in Krainer et al. (Microb Cell Fact. 2015. 14:4) was sterilized in 1 L glass bioreactor vessels (DASGIP, Switzerland) prior to inoculation. A batch phase of 12 h was performed at 28 °C, a minimum saturation of 30% of dissolved oxygen throughout the cultivation was controlled by a cascade setting for stirrer speed, air flow and oxygen flow. Thirteen hours after inoculation, a linearly increasing glycerol feed rate starting at 6.85 g/L/h was initiated for 17 h to a final max. glycerol feed rate of 8.72 g/L/h. Then, the feed rate was set to facilitate a constant growth rate of 0.014 /h, and the temperature was set to 24 °C. This feed rate was continued until harvest at 120 hours post inoculation. The feed medium contained 696 g/L glycerol, 4.4 g/L Trace Element Solution (Krainer et al. Microb Cell Fact. 2015. 14:4), 200 ppm biotin and 0.3 mL/kg antifoam Struktol J650. Supernatants were regularly sampled by centrifugation of the culture broth at 4000 rpm in a bench top centrifuge (Eppendorf Centrifuge 5810R, Eppendorf, Germany) and further used for enzymatic activity determination.

The amounts of reporter signal produced by a strain harboring an expression cassette comprising the non-truncated *FMD* promoter, i.e. the promoter having the nucleotide sequence of SEQ ID NO: 1, throughout the cultivation are shown in Fig. 8. After approximately 119 h of cultivation, the obtained volumetric enzyme activity was set to 100% as reporter signal. A strain harboring an expression cassette comprising a promoter truncated from the 5'-end of the nucleotide sequence of SEQ ID NO: 1 to a length of 280 bp produced a reporter signal of 115% after approximately 119 hours of cultivation (Fig. 9). Several different strains harboring an expression cassette comprising a promoter truncated from the 5'-end of the nucleotide sequence of SEQ ID NO: 1 to a length of 160 bp were cultivated and produced reporter signals of 112.6%, 160.4%, 139.1%, 149.2%, 107.0%, 110.1% and 134.5% after approximately 119 h of cultivation (Fig. 10). Concluding, the 623 bp promoter had a relative expression efficiency (REE) of 0.161 %/bp, the 280 bp promoter had a REE of 0.411 %/bp, and the 160 bp promoter had a REE between 0.699 and 1.003 %/bp. Thus, the REE of the 280 bp promoter was found to be 2.56-fold the REE of the non-truncated 623 bp promoter, and the REE of the 160 bp promoter was found to be 4.16-6.25-fold the REE of the 623 bp promoter. Surprisingly, all strains harboring a truncated promoter were found to produce more target enzyme than the non-truncated promoter.

### Example 4: Promoter induction by glycerol or glucose feed

In WO 2017/109082 A1 and in Vogl et al. (AMB Express. 2020. 10(1): 38) it is taught that in P. *pastoris*, expression from a 623 bp wildtype *HpFMD* promoter is activated, i.e. induced, upon depletion of glucose or glycerol. To study, whether truncated promoters as described herein would follow this dogma, both, actual depletion of glucose/glycerol by no feed, as well as different constant glucose or glycerol feed rates ranging from 0.07 mmol glycerol/g CDW/h or 0.04 mmol glucose/g CDW/h to 0.48 mmol glycerol/g CDW/h or 0.25 mmol glucose/g CDW/h were tested, after the linearly increasing glycerol feed rate starting at 6.85 g/L/h was performed for 17 h as described in Example 3, i.e. 30 hours after cultivation start. Otherwise, the cultivations were performed essentially as described in Example 3. In addition, these conditions were also tested for the non-truncated wildtype *FMD* promoter. As reporter enzyme, the esterase enzyme of Example 1 was used.

Reporter signals from the produced reporter enzyme obtained from different glucose or glycerol feed rates as described above are shown in Table 4 as examples wherein expression was regulated by a truncated *FMD* promoter having 280 bp.

**Table 4: Volumetric enzyme activities obtained after approximately 60 h of cultivation, when promoter induction was facilitated either via depletion of glucose/glycerol (i.e. 0.00 mmol/g CDW/h) or under feeding different rates of glucose or glycerol. The reporter signal obtained under glucose depletion was set to 100%**

| **Glucose feed rate mmol Glu/g CDW/h** | **Glycerol feed rate mmol Gly/g CDW/h** | **Reporter signal %** |
|---|---|---|
| 0.00 | - | 100.0 |
| - | 0.00 | 102.8 |
| 0.04 | - | 141.7 |
| - | 0.07 | 135.0 |
| 0.11 | - | 168.8 |
| - | 0.21 | 151.9 |
| 0.18 | - | 182.8 |
| - | 0.35 | 223.3 |
| 0.25 | - | 249.6 |
| - | 0.48 | 301.2 |

Unexpectedly, even a positive correlation between feed rate and specific productivity could be observed: Higher feed rates resulted in higher volumetric and specific activity. The same trend was found when expression was regulated either by a 623 bp promoter, as well as when expression was regulated by truncated promoter variants having 500 bp, 350 bp, 280 bp or 160 bp. Based thereon, it was concluded that surprisingly and in contrast to previous publications the best mode of operating the wildtype *FMD* promoter or truncated variants thereof in P. *pastoris* was not via activation by depletion of glucose or glycerol, but by feeding at least 0.07 mmol Gly/g CDW/h or 0.04 mmol Glu/g CDW/h.

### Example 5: Recombinant gene expression with truncated eukaryotic promoter in Yarrowia lipolytic

To test whether the expression via truncated FMD promoters would be limited to P. *pastoris* strains (as found for *K*. *phaffii* CBS 7435, *K. phaffii* GS115 and *K. pastoris* CBS 704), *Yarrowia lipolytica* was tested. Transformation of a *Y. lipolytica* CBS 7504 strain with an expression cassettes as described in Example 1 was performed by electroporation according to the protocol of Lin-Cereghino et al. (Biotechniques. 2005. 38(1): 44-48). Notwithstanding, alternative protocols for transformation of *Y. lipolytica* might be employed to transform this yeast with expression cassettes. Subsequently, cells were incubated and selected on agar plates containing suitable antibiotics for selecting positive transformants. The cultivation of at least 50 positive transformants was performed in 96-well plates essentially as described in Weis et al. (FEMS Yeast Res. 2004. 5(2): 179-189): Cell growth was enabled in 450 µL YPD (1% w/v yeast extract, 2% w/v peptone, 2% w/v glucose) per well at 28 °C, 320 rpm at 90% humidity in a Kuhner ISF1-X Climo-Shaker for 60 hours, analogous to Example 1. After incubation for 60 hours at 320 rpm and 28 °C, cells were fed twice with 100 µL of YPD per well, 60 h and 85 h after inoculation, and then cultivated for additional 24 h before harvesting by centrifugation. Reporter protein levels in the supernatant were determined by monitoring volumetric esterase activity of the hydrolysis of 4-nitrophenyl 2-(trimethylsilyl)ethyl carbonate. The hydrolysis reaction was followed by photometrically measuring the increase in absorbance at 405 nm essentially as described in Ruth et al. (Microb Cell Fact. 2014. 13: 120). Analogous to the recombinant production controlled by truncated *FMD* promoters in *P. pastoris* strains, essentially the same increase in REE was found when these promoters were used to regulate recombinant protein production in *Y. lipolytica.* It was found, that not only *P*. *pastoris,* but also *Y*. *lipolytica* showed the ability to produce and secrete target reporter protein, whose production was controlled by truncated *FMD* promoters.

## Claims

1. Promoter, **characterized in that**
the promoter is truncated from the 5'-end of the nucleotide sequence of SEQ ID NO: 1 to a length of 140-610 base pairs, preferably to a length of 140-560 base pairs, more preferably to a length of 160-560 base pairs, most preferably to a length of 160-500 base pairs;
or promoter variant, wherein the promoter variant has at least 80% identity, preferably at least 90% identity, more preferably at least 95% identity, more preferably at least 97% identity, most preferably at least 99% identity to the promoter.

2. The promoter or promoter variant of claim 1, wherein the relative expression efficiency of the promoter or promoter variant is at least 5%, preferably at least 10% higher than the relative expression efficiency of the promoter having the nucleotide sequence of SEQ ID NO: 1.

3. The promoter or promoter variant according to any one of claims 1 and 2, wherein the promoter or promoter variant is comprised in an expression cassette, wherein the expression cassette further comprises a nucleotide sequence coding for a polypeptide and optionally a transcription terminator sequence, and wherein the promoter or promoter variant is operably linked to the nucleotide sequence coding for a polypeptide.

4. The promoter or promoter variant according to any one of claims 1 and 2, wherein the promoter or promoter variant is comprised in a plasmid, and wherein the promoter or promoter variant is operably linked to a nucleotide sequence coding for a polypeptide.

5. The promoter or promoter variant according to any one of claims 1 and 2, wherein the promoter or promoter variant is comprised in a yeast cell, and wherein the promoter or promoter variant is operably linked to a nucleotide sequence coding for a polypeptide.

6. The promoter or promoter variant according to claim 5, wherein the yeast cell is a cell of a genus selected from the group consisting of *Pichia*, *Komagataella*, *Ogataea*, *Candida*, *Hansenula*, *Saccharomyces*, *Schizosaccharomyces*, *Kluyveromyces*, *Zygosaccharomyces* and *Yarrowia*, preferably a *Pichia pastoris* cell or a *Yarrowia lipolytica* cell, most preferably a *Pichia pastoris* cell.

7. Method for recombinant production of a target polypeptide comprising the steps of
a) introducing an expression cassette comprising a nucleotide sequence encoding the target polypeptide into at least one yeast cell;
b) producing a yeast cell culture by multiplying the at least one yeast cell;
c) subjecting the yeast cell culture to a condition suitable for expression of the nucleotide sequence encoding the target polypeptide; and
d) isolating the produced target protein from the yeast cell culture,
**characterized in that**
the expression cassette further comprises a promoter or promoter variant according to any one of claims 1 or 2 operably linked to the nucleotide sequence encoding the target polypeptide.

8. Method according to claim 7, **characterized in that** the condition suitable for expression of the nucleotide sequence encoding the target polypeptide is a fed-batch process.

9. Method according to claim 8, **characterized in that** the fed-batch process is operated with a glucose feed rate of at least 0.04 mmol of glucose per gram of cell dry weight and per hour (mmol Glu/g CDW/h) or a glycerol feed rate of at least 0.07 mmol of glycerol per gram of cell dry weight and per hour (mmol Gly/g CDW/h), preferably with a glucose feed rate of at least 0.11 mmol Glu/g CDW/h or a glycerol feed rate of at least 0.21 mmol Gly/g CDW/h, more preferably with a glucose feed rate of at least 0.18 mmol Glu/g CDW/h or a glycerol feed rate of at least 0.35 mmol Gly/g CDW/h.

10. Method according to any one of claims 7, 8 or 9, wherein the yeast cell is a *Pichia pastoris* cell or a *Yarrowia lipolytica* cell, preferably a *Pichia pastoris* cell.

11. Method for increasing the relative expression efficiency of a nucleotide sequence having promoter activity,
the method comprising the step of providing a truncated promoter by truncating a nucleotide sequence having at least 80%, preferably at least 90% identity, more preferably at least 95% identity, more preferably at least 97% identity, more preferably at least 98% identity, even more preferably at least 99% identity, most preferably 100% identity to the nucleotide sequence of SEQ ID NO: 1 from the 5'-end to a length of 140-610 base pairs, preferably to a length of 140-560 base pairs, more preferably to a length of 160-560 base pairs, most preferably to a length of 160-500 base pairs.

12. Method according to claim 11, wherein the relative expression efficiency of the truncated promoter is at least 5%, preferably at least 10%, more preferably at least 20%, even more preferably at least 50%, most preferably at least 100% higher than the relative expression efficiency of the promoter having the nucleotide sequence of SEQ ID NO: 1.
